# EUROPÄISCHE PATENTANMELDUNG

(11) **EP 3 651 161 A1**
(43) Veröffentlichungstag der Anmeldung: **13.05.2020**
(21) Anmeldenummer: 18204937.9
(22) Anmeldetag: 07.11.2018
(51) Int. Cl.: G16H 40/63, G16H 30/20, G01R 33/54, A61B 6/00

(54) **VERFAHREN, SYSTEM UND MEDIZINISCHES BILDGEBUNGSSYSTEM ZUR ERSTELLUNG EINES ABBILDES VON EINEM UNTERSUCHUNGSOBJEKT SOWIE VERWENDUNG DERARTIGER ABBILDER**

(71) Anmelder: Siemens Healthcare GmbH, 91052 Erlangen (DE)
(72) Erfinder: Feiweier, Thorsten, 91099 Poxdorf (DE); Prinz, Carsten, 91083 Baiersdorf (DE); Schneider, Michael, 91056 Erlangen (DE); Zenge, Michael, 90419 Nürnberg (DE)

(57) **Zusammenfassung**

Die Erfindung betrifft ein Bildgebungssystem zur Erstellung eines Abbildes von einem Untersuchungsobjekt, welches eine Systemkomponente und eine Steuerungskomponente aufweist. Die Systemkomponente hat eine Komponenteneigenschaft, welche einen Wert in einem durch das Bildgebungssystem bedingten ersten Wertebereich einnehmen kann. Dabei weist eine entsprechende Systemkomponente eines anderen Bildgebungssystems eine entsprechende Komponenteneigenschaft mit einem anderen Wertebereich auf, der mit dem ersten Wertebereich einen Überlappungsbereich aufweist. Die Steuerungskomponente ist ausgebildet, eine Verwendung der Systemkomponente bei Erstellung des Abbildes zu steuern, wobei die Steuerungskomponente in einem Kompatibilitätsmodus betreibbar ist. Im Kompatibilitätsmodus ist die Steuerungskomponente eingerichtet, bei Erstellung des Abbildes nur Werte für die Komponenteneigenschaft der Systemkomponente zuzulassen, die in dem Überlappungsbereich liegen.

## Beschreibung

Die Erfindung liegt auf dem Gebiet der Bildgebung und betrifft insbesondere ein Bildgebungssystem zur Erstellung eines Abbildes von einem Untersuchungsobjekt, ein medizinisches Bildgebungssystem, ein Verfahren zur Bestimmung eines Abbildes von einem Untersuchungsobjekt sowie die Verwendung von derartig erstellten Abbildern.

Üblicherweise werden zur Erstellung eines Abbildes von einem Untersuchungsobjekt, etwa einem Patienten in der medizinischen Bildgebung, Systemkomponenten eines Bildgebungssystems gesteuert, so dass Komponenteneigenschaften von den Systemkomponenten bestimmte Werte einnehmen. Dabei ist das erstellte Abbild von dem jeweiligen Bildgebungssystem und den jeweils eingestellten Werten abhängig. Insbesondere bei der medizinischen Bildgebung nimmt die Anzahl an unterschiedlichen Typen von bildgebenden Medizingeräten - wie verschiedene Computertomografie- oder Magnetresonanztomographieanlagen - weiterhin zu. Hierdurch lassen sich verschiedenen Kundensegmenten an die jeweiligen Anforderungen angepasste Geräte/Systeme anbieten. Differenzierungsmerkmale können dabei Performance- oder Ausstattungsmerkmale sein, welche bestimmten Komponenteneigenschaften und Werten, welche die jeweilige Komponenteneigenschaft einnehmen kann, entsprechen. Typischerweise werden MR-Anlagen (Magnetresonanztomographieanlagen) mit unterschiedlicher Grundfeldstärke, unterschiedlicher Gradientenfeldstärke und -schaltgeschwindigkeit sowie unterschiedlicher Anzahl von Sende- oder Empfangskanälen angeboten.

Um die gewünschte diagnostische Qualität zu liefern - etwa Kontrast, Auflösung, Signal-zu-Rausch-Verhältnis etc. - oder möglichst kurze Aufnahmezeiten zu ermöglichen, werden die für das jeweilige Bildgebungssystem ausgelieferten Messprotokolle gewöhnlich optimiert. So kann etwa durch Ausreizen der verfügbaren Performance-Merkmale - also insbesondere das Verwenden von Komponenteneigenschaften mit Werten, die für die jeweilige Anwendung optimiert sind - die Aufnahmezeit verkürzt werden. So lässt sich etwa bei einer MR-Anlage mit einem performanteren, d. h. leistungsfähigeren Gradienten- oder Empfangssystem durch höhere Gradienten - also Setzen der Gradientenwerte in einem höheren Bereich bei dem Gradientensystem - in kürzerer Zeit ein Abbild mit einer vergleichbaren Bildqualität erstellen werden als bei einer anderen MR-Anlage mit einem weniger leistungsfähigen Gradientensystem.

Allerdings können die optimierten Messprotokolle dazu führen, dass Abbilder, die mit unterschiedlichen Bildgebungssystemen erstellt wurden, etwa bei Nachfolgeuntersuchungen nicht direkt miteinander vergleichbar sind und aufgrund individueller Optimierungen der Messprotokolle - etwa für eine MR-Anlage und eine andere MR-Anlage - ein unterschiedlicher Bildeindruck entstehen kann.

Es besteht Bedarf, die Bildgebung zu verbessern und dabei insbesondere eine Reproduzierbarkeit eines Bildeindrucks, also von Eigenschaften eines mittels Bildgebung erstellten Abbildes - etwa für Nachfolgeuntersuchungen in der medizinischen Bildgebung - zu steigern und/oder eine Benutzung von mehreren etwaig unterschiedlichen Bildgebungssystemen effizienter zu machen.

Die Erfindung löst die Aufgabe jeweils durch ein Bildgebungssystem zur Erstellung eines Abbildes von einem Untersuchungsobjekt, durch ein medizinisches Bildgebungssystem, durch ein Verfahren zur Bestimmung eines Abbildes von einem Untersuchungsobjekt und durch eine Verwendung davon je gemäß der Lehre eines der Hauptansprüche. Vorteilhafte Ausführungsformen, Weiterbildungen und Varianten der vorliegenden Erfindung sind insbesondere Gegenstand der Unteransprüche.

Ein erster Aspekt der Erfindung betrifft ein Bildgebungssystem zur Erstellung eines Abbildes von einem Untersuchungsobjekt. Das Bildgebungssystem weist eine Systemkomponente und eine Steuerungskomponente auf. Die Systemkomponente hat eine Komponenteneigenschaft, welche bei Erstellung des Abbildes einen Wert in einem durch das Bildgebungssystem bedingten ersten Wertebereich einnehmen kann. Dabei weist eine entsprechende Systemkomponente eines anderen Bildgebungssystems eine entsprechende Komponenteneigenschaft mit einem anderen Wertebereich auf, der mit dem ersten Wertebereich einen Überlappungsbereich aufweist. Die Steuerungskomponente ist ausgebildet, eine Verwendung der Systemkomponente bei Erstellung des Abbildes zu steuern, wobei die Steuerungskomponente in einem Kompatibilitätsmodus betreibbar ist. Im Kompatibilitätsmodus ist die Steuerungskomponente eingerichtet, bei Erstellung des Abbildes nur Werte für die Komponenteneigenschaft der Systemkomponente zuzulassen, die in dem Überlappungsbereich liegen.

Im Sinne der Erfindung ist unter einer "Komponenteneigenschaft" zumindest eine physische Eigenschaft oder eine funktionelle Eigenschaft zu verstehen. So kann etwa die Systemkomponente eine bestimmte Funktion im/über den ersten Wertebereich ausführen und dabei einen physischen Parameter bei der Erstellung des Abbildes einstellen und/oder einen bestimmten Werte für eine physikalische Größe, wie etwa eine Feldstärke, herbeiführen. Auch kann etwa die Systemkomponente eine bestimmte Funktion anhand von Software ausführen, wobei bestimmte Implementierungen bei einem Bildgebungssystem oder bei einem anderen Bildgebungssystem vorhanden oder nichtvorhanden sein können, sodass das eine Bildgebungssystem die bestimmte Implementierung bereitstellt, während das andere Bildgebungssystem diese Implementierung nicht bereitstellt (bzw. nicht bereitstellen kann). Dabei können solche Software-Implementierung oder -Funktionen jeweils durch einen Wert gekennzeichnet werden, sodass die jeweils davon vorhandenen - oder auch möglichen - einen Wertebereich definieren und/oder umgekehrt ein Ausführen der Funktionen oder Implementierungen auf eine Untermenge, etwa in einem Überlappungsbereich von Wertebereichen, eingeschränkt werden kann.

Ein Vorteil des Zulassens von Werten für die Komponenteneigenschaft nur in dem Überlappungsbereich kann insbesondere darin liegen, dass auch die entsprechende Komponenteneigenschaft des anderen Bildgebungssystems solche Werte einnehmen kann. So lässt sich ein Messprotokoll, welches ein Einstellen derartiger Werte bewirkt, auf beiden Systemen, also auf dem Bildgebungssystem und dem anderen Bildgebungssystem ausführen, wodurch sich die Benutzung von den Bildgebungssystemen effizienter machen lässt. Außerdem lässt sich ein vergleichbarer Bildeindruck auf beiden Bildgebungssystemen erzielen, indem gleiche Werte für die Komponenteneigenschaft eingestellt werden. Folglich lässt sich das gleiche Messprotokoll auf den beiden unterschiedlichen Bildgebungssystemen ausführen- auch ohne etwaige vorhergehende Konvertierung -, wodurch sich die unterschiedlichen Bildgebungssysteme abhängig von ihrer jeweiligen Verfügbarkeit und damit effizient einsetzen lassen, und es lassen sich konsistente Abbilder des Untersuchungsobjektes mit den beiden unterschiedlichen Bildgebungssystemen erzeugen und somit ein konsistenter Bildeindruck erzielen, wodurch etwa eine auf solchen Abbildern basierende Diagnose bei der medizinischen Bildgebung verbessert und/oder erleichtert werden kann.

Gemäß einigen Ausführungsformen weist das Bildgebungssystem eine Eingabeeinrichtung auf, die eingerichtet ist, basierend auf einer Eingabe eines Benutzers den Kompatibilitätsmodus zu aktivieren oder zu deaktivieren.

Gemäß einigen Ausführungsformen weist das Bildgebungssystem eine Ausgabeeinrichtung auf, die eingerichtet ist, auszugeben, insbesondere mittels einer Benutzerschnittstelle, ob der Kompatibilitätsmodus aktiviert ist oder nicht. Auf diese vorteilhafte Weise kann ein Benutzer erkennen, etwa bevor er ein Messprotokoll ausführen lässt, ob der Kompatibilitätsmodus aktiv ist oder welcher - bei etwaig mehreren Kompatibilitätsmodi - Kompatibilitätsmodus aktiv ist.

Gemäß einigen Ausführungsformen ist die Steuerungskomponente in dem Kompatibilitätsmodus oder in einem Leistungsmodus betreibbar. Dabei ist die Steuerungskomponente im Leistungsmodus eingerichtet, um bei Erstellung des Abbildes im Leistungsmodus Werte der Komponenteneigenschaft der Systemkomponente zuzulassen, die im ersten Wertebereich liegen aber außerhalb des Überlappungsbereichs. Hierdurch lässt sich eine Qualität der Abbildung steigern und/oder eine erforderliche Aufnahmezeit zur Erstellung des Abbildes gegenüber der Erstellung des Abbildes im Kompatibilitätsmodus reduzieren.

Gemäß einigen Ausführungsformen ist die Steuerungskomponente in dem Kompatibilitätsmodus oder in einem Leistungsmodus betreibbar. Dabei ist die Steuerungskomponente im Leistungsmodus eingerichtet, um bei Erstellung des Abbildes im Leistungsmodus Werte der Komponenteneigenschaft der Systemkomponente zuzulassen, die im ersten Wertebereich liegen aber nicht notwendigerweise außerhalb des Überlappungsbereichs. Hierdurch werden Messprotokolle ermöglicht, die einen größeren Bereich der möglichen Werte für die Komponenteneigenschaft verwenden, sodass etwa ein Benutzer des Bildgebungssystems im Leistungsmodus mehr Freiheiten beim Benutzen des Bildgebungssystems hat und es ermöglicht wird, die Leistungsfähigkeit des Bildgebungssystems weiter auszureizen.
Bei einigen Ausführungsformen, bei welchen die Steuerungskomponente in einem Leistungsmodus betreibbar ist, ist die Steuerungskomponente im Leistungsmodus weiterhin eingerichtet, ein Messprotokoll für die Erstellung des Abbildes für den Kompatibilitätsmodus in ein Messprotokoll für den Leistungsmodus zu konvertieren und das konvertierte Messprotokoll auszuführen. Auf diese vorteilhafte Weise lassen sich Messprotokolle für den Kompatibilitätsmodus auch im Leistungsmodus ausführen. Dabei ist die Steuerungskomponente in einigen Varianten eingerichtet, um bei der Konvertierung oder bei der Ausführung des konvertierten Messprotokolls auch Werte für die Komponenteneigenschaft zu verwenden, welche außerhalb des Überlappungsbereichs liegen, so dass die etwaig höhere Leistungsfähigkeit im Leistungsmodus auch bei konvertierten Messprotokollen verwendet werden kann.

Gemäß einigen Ausführungsformen können bei einer Konvertierung eines Messprotokolls eine oder mehrere Parameter des Messprotokolls an das jeweilige Bildgebungssystem oder an den jeweiligen Kompatibilitätsmodus angepasst werden.

Gemäß einiger Ausführungsformen ist die Steuerungskomponente weiterhin eingerichtet, ein Messprotokoll, welches ursprünglich nicht für den Kompatibilitätsmodus erstellt wurde - etwa ein Messprotokoll für einen Leistungsmodus - in ein Messprotokoll für den Kompatibilitätsmodus zu konvertieren und das konvertierte Messprotokoll im Kompatibilitätsmodus auszuführen. Hierdurch lassen sich weitere Messprotokolle im Kompatibilitätsmodus verwenden, wodurch die Effizienz beim Betreiben von solchen Bildgebungsgeräten gesteigert werden kann. Außerdem ist in einigen Varianten die Steuerungskomponente eingerichtet, das konvertierte Messprotokoll zu speichern, wodurch es im Kompatibilitätsmodus auf dem Bildgebungssystem und dem anderen Bildgebungssystem, insbesondere ohne zusätzlicher/erneuter Konvertierung, ausgeführt werden kann.

Zur Konvertierung eines Messprotokolls für den Kompatibilitätsmodus in ein Messprotokoll in einen anderen Betriebsmodus, insbesondere den Leistungsmodus, oder für eine Konvertierung in umgekehrter Richtung kann die Steuerungskomponente in einigen Varianten eingerichtet sein, eine Protokollkonvertierungsfunktion auszuführen. Dabei kann die Protokollkonvertierungsfunktion in einigen Varianten einer sogenannten "Phoenix-Funktion" entsprechen.

Durch die Protokollkonvertierungsfunktion können Werte von einer Kombination an Systemkomponenten in für den jeweils anderen Betriebsmodus geeignete Werte automatisch umgewandelt werden. So können etwa bei einer MR-Anlage geringere erreichbare Werte für eine Gradientenstärke (also insbesondere Gradientenfeldstärke bzw. Magnetfeldstärke) und/oder eine Gradientenanstiegsrate (also insbesondere kurz Anstiegsrate) durch entsprechend längere Anstiegszeiten oder Haltedauern zumindest teilweise ausgeglichen werden. Da jedoch ein tatsächlicher Messablauf aufgrund des angepassten Messprotokolls, also des konvertierten Messprotokolls, unterschiedlich von einem tatsächlichen Messablauf mit dem ursprünglichen Messprotokoll ist, können sich Unterschiede in der Erstellung des Abbildes und somit ein unterschiedlicher Bildeindruck ergeben. Dennoch kann in der Konvertierung insbesondere ein Vorteil darin liegen, dass ein Messprotokoll auf verschiedenen Bildgebungssystemen verwendet werden kann, wobei die automatische Konvertierung eine effiziente Verwendung von unterschiedlichen Bildgebungsgeräten ermöglicht und/oder den resultierenden Bildeindruck, soweit aufgrund der unterschiedlichen Systemkomponenten möglich, ähnlich hält.

Im Rahmen der Offenbarung wird als eine sogenannte "Phoenix-Funktion" insbesondere oder lediglich eine Funktionalität bezeichnet, ein Messprotokoll von einem ersten Bildgebungssystem auf ein anderes Bildgebungssystems übertragen. Sofern die Systemkomponenten des anderen Bildgebungssystems es erlauben, das Protokoll ohne Änderungen auszuführen, wird das Protokoll unverändert verwendet, andernfalls wird das Protokoll automatisch an die Leistungsfähigkeit der Systemkomponenten des anderen Bildgebungssystems angepasst.

Im Unterschied zu einer sogenannten "Phoenix-Funktion" ermöglicht es der Kompatibilitätsmodus, auf unterschiedlichen Bildgebungssystemen vergleichbare - oder sogar gleiche - Bildeindrücke beim Erstellen eines Abbildes von einem Untersuchungsobjekt zu erzielen, da nur Werte für die Komponenteneigenschaft der Systemkomponente zugelassen werden, welche für die betreffenden Bildgebungssysteme im Überlappungsbereich, also gemeinsam möglich sind und folglich beim Erstellen des Abbildes im Kompatibilitätsmodus auch nur solche Werte zugelassen bzw. bzgl. der Komponenteneigenschaft eingestellt werden. Auf diese Weise ist bei einigen bevorzugten Varianten keinerlei Konvertierung der einzustellenden Werte für die Systemkomponente erforderlich, da der (gemeinsame) Kompatibilitätsmodus garantiert, dass bei einer Übertragung eines solchen Messprotokolls für den Kompatibilitätsmodus zwischen zwei Bildgebungssysteme mit diesem Kompatibilitätsmodus bzw. der gleichen Kompatibilitätsklasse keine Anpassung des Messprotokolls erforderlich ist.

Gemäß einigen Ausführungsformen ist die Steuerungskomponente weiterhin eingerichtet, zu überprüfen, ob bei Erstellung des Abbildes die einzustellenden Werte für die Komponenteneigenschaft im ersten Wertebereich und/oder zumindest im Kompatibilitätsmodus auch im Überlappungsbereich liegen. Dabei wird die Erstellung des Abbildes in Abhängigkeit von einem Ergebnis der Überprüfung durchgeführt oder nicht und/oder, sofern die Werte zwar im ersten Wertebereich aber außerhalb des Überlappungsbereichs liegen, das Abbild in einem Leistungsmodus erstellt wird und ein Fehlersignal ausgegeben wird. Falls die Erstellung nicht durchgeführt wird, da die einzustellenden Werte außerhalb des ersten Wertebereichs oder außerhalb des Überlappungsbereichs liegen, ist die Steuerungskomponente in einigen Varianten eingerichtet, ein Fehlersignal bereitzustellen.

Bei einigen Ausführungsformen legt der Kompatibilitätsmodus ein erstes systemunabhängiges Bildgebungssystem mit einer systemunabhängigen Systemkomponente und einer systemunabhängigen Steuerungskomponente fest. Die systemunabhängige Systemkomponente weist eine Komponenteneigenschaft auf, welche mit der Komponenteneigenschaft der Systemkomponente des Bildgebungssystems korrespondiert und einen Wert in einem systemunabhängigen Wertebereich einnehmen kann, der vollständig im Überlappungsbereich liegt. Die systemunabhängige Steuerungskomponente ist eingerichtet, ein Messprotokoll für den Kompatibilitätsmodus auszuführen und dabei den Wert der Komponenteneigenschaft der systemunabhängigen Systemkomponente auf einen oder mehrere Werte im systemunabhängigen Wertebereich einzustellen. Die Steuerungskomponente des Bildgebungssystems ist im Kompatibilitätsmodus eingerichtet, ein Messprotokoll für die Erstellung des Abbildes für den Kompatibilitätsmodus der systemunabhängigen Steuerungskomponente zur Ausführung bereitzustellen und den Wert der Komponenteneigenschaft der Systemkomponente anhand des Werts der Komponenteneigenschaft der systemunabhängigen Systemkomponente einzustellen. In einigen Varianten können die systemunabhängige Systemkomponente und/oder die systemunabhängige Steuerungskomponente virtualisierte Komponenten sein, welche in einer Softwareimplementierung physische Komponenten abstrahieren, so dass insbesondere für eine softwareseitige Steuerung - etwa anhand eines Messprotokolls - Komponenten von unterschiedlichen physischen Bildgebungssystemen - also etwa die Systemkomponente und die Steuerungskomponente des Bildgebungssystems gemäß dem ersten Aspekt der Erfindung - gleich repräsentiert sind. Auch kann in einigen Varianten die Steuerungskomponente des Bildgebungssystems eingerichtet sein, eine virtuelle Maschine für die systemunabhängige Steuerungskomponente und/oder die systemunabhängige Systemkomponente auszuführen. Durch einen solchen Kompatibilitätsmodus mit systemunabhängigen Komponenten lassen sich vorteilhaft Funktionen zur Steuerung des Bildgebungssystems (und weiterer Bildgebungssysteme) systemunabhängig implementieren, wodurch insbesondere die Effizienz beim Einsatz von mehreren, insbesondere unterschiedlichen, Bildgebungssystemen gesteigert werden kann und/oder die Reproduzierbarkeit beim Erstellen von Abbildern erhöht werden kann.

Bei einigen Ausführungsformen, bei welchen der Kompatibilitätsmodus eine systemunabhängige Systemkomponente festlegt, ist die systemunabhängige Systemkomponente eingerichtet, eine Schnittstelle zwischen der systemunabhängigen Steuerungskomponente und der Systemkomponente des Bildgebungssystems bereitzustellen und dabei die Werte der Systemkomponente und der systemunabhängigen Systemkomponente ineinander umzuwandeln. Zudem ist die Steuerungskomponente eingerichtet, Werte der Komponenteneigenschaft der Systemkomponente des Bildgebungssystems auf Basis der systemunabhängigen Systemkomponente einzustellen. Auf diese vorteilhafte Weise lässt sich die Systemkomponente des Bildgebungssystems, also eine physische Komponente, über die systemunabhängige Systemkomponente steuern, wobei in einigen Varianten die Werte für die Komponenteneigenschaft der systemunabhängigen Systemkomponente von den Werten der Komponenteneigenschaft der (physischen) Systemkomponente abstrahiert sein können. Hierdurch lässt sich eine langfristige Kompatibilität zwischen verschiedenen Generationen an Bildgebungssystemen erzielen.

Gemäß einigen Ausführungsformen ist die Steuerungskomponente im Kompatibilitätsmodus für eine erste Systemklasse, die das Bildgebungssystem und das andere Bildgebungssystem umfasst, und in einem Kompatibilitätsmodus für eine zweite Systemklasse, die zumindest noch ein anderes Bildgebungssystem umfasst, betreibbar. Dabei weist eine entsprechende Systemkomponente des noch einen anderen Bildgebungssystems eine entsprechende Komponenteneigenschaft mit einem zweiten Wertebereich auf, so dass die Komponenteneigenschaft der entsprechenden Systemkomponente des noch einen anderen Bildgebungssystems einen Wert in dem zweiten Wertebereich einnehmen kann, wobei der zweite Wertebereich durch das noch eine andere Bildgebungssystem bedingt ist. Ein Vorteil der beiden Kompatibilitätsmodi kann insbesondere darin liegen, dass unterschiedliche Bildgebungssysteme jeweils einem oder mehreren der Kompatibilitätsmodi zugeordnet werden können und sich somit mittels der Steuerungskomponente jeweils in einem solchen Kompatibilitätsmodus betreiben lassen, dessen Überlappungsbereich bzw. Wertebereich Werte definiert, die die jeweilige Komponenteneigenschaft der jeweiligen Systemkomponente auch tatsächlich einnehmen kann. Auch können in einigen Varianten Messprotokolle jeweils für die Kompatibilitätsmodi erstellt und/oder optimiert oder von einem der Kompatibilitätsmodi in den jeweils anderen konvertiert werden.

Bei einigen Ausführungsformen, bei welchen die Steuerungskomponente in einem Kompatibilitätsmodus für eine zweite Systemklasse betreibbar ist, sind der Überlappungsbereich und der zweite Wertebereich disjunkt oder überlappen nur teilweise.

Bei einigen Ausführungsformen, bei welchen die Steuerungskomponente in einem Kompatibilitätsmodus für eine zweite Systemklasse betreibbar ist, ist die Steuerungskomponente eingerichtet, bei Erstellung des Abbildes Werte für die Komponenteneigenschaft, welche im zweiten Wertebereich aber außerhalb des Überlappungsbereichs liegen, in solche Werte zu konvertieren, die im Überlappungsbereich liegen.

Bei einigen Ausführungsformen, bei welchen der Überlappungsbereich und der zweite Wertebereich nur teilweise überlappen, ist die Steuerungskomponente eingerichtet, bei Erstellung des Abbildes im Kompatibilitätsmodus für die erste Systemklasse nur Werte für die Komponenteneigenschaft der Systemkomponente zuzulassen, die in dem Überlappungsbereich liegen. Zusätzlich oder alternativ ist die Steuerungskomponente eingerichtet, bei Erstellung des Abbildes im Kompatibilitätsmodus für die zweite Systemklasse nur Werte für die Komponenteneigenschaft der Systemkomponente zuzulassen, die in dem ersten Wertebereich und zugleich in dem zweiten Wertebereich liegen.

Bei einigen Ausführungsformen, bei welchen die Steuerungskomponente in einem Kompatibilitätsmodus für eine zweite Systemklasse betreibbar ist, liegt der zweite Wertebereich, insbesondere vollständig, im Überlappungsbereich - also jenem Wertebereich, in welchem sich die Wertebereiche für die Komponenteneigenschaften der Systemkomponenten der ersten Systemklasse überlappen. Auf diese vorteilhafte Weise besteht mit dem Kompatibilitätsmodus für die erste Systemklasse ein Kompatibilitätsmodus für leistungsfähigere Bildgebungssysteme, die also einen größeren Wertebereich ermöglichen, während durch den Kompatibilitätsmodus für die zweite Systemklasse ein zusätzlicher Kompatibilitätsmodus mit Bildgebungssystemen mit geringerer Leistungsfähigkeit, welche also nur einen kleineren Wertebereich ermöglichen, bereitgestellt wird.

Bei einigen Ausführungsformen, bei welchen die Steuerungskomponente im Kompatibilitätsmodus für eine erste Systemklasse und im Kompatibilitätsmodus für eine zweite Systemklasse betreibbar ist, umfasst sowohl die erste als auch die zweite Systemklasse das Bildgebungssystem gemäß dem ersten Aspekt der Erfindung und das andere Bildgebungssystem, während das noch eine andere Bildgebungssystem von der ersten Systemklasse nicht umfasst ist. Durch den Kompatibilitätsmodus für die zweite Systemklasse lässt sich also eine Kompatibilität und somit ein gleicher Bildeindruck von Abbildern, die über den Kompatibilitätsmodus für die zweite Systemklasse erstellt worden sind, für eine größere Anzahl an Bildgebungssysteme erzielen, als für den Kompatibilitätsmodus für die erste Systemklasse, welche etwaig Bildgebungssysteme mit höherer Leistungsfähigkeit umfasst.

Ein Vorteil der Kompatibilitätsmodi und insbesondere von einer Abstufung an Systemklassen, welche zunehmend weitere Bildgebungssysteme umfassen, kann insbesondere darin liegen, dass Messprotokolle jeweils für einen noch geeigneten Kompatibilitätsmodus, welcher jedoch die verhältnismäßig geringsten Anforderungen an die Leistungsfähigkeit der Bildgebungsgeräte stellt, erstellt werden können, so dass über eine große Anzahl an Bildgebungssystemen ein gleicher Bildeindruck erzielt werden kann und/oder sich die Effizienz beim Erstellen von Messprotokollen steigern lässt. Während also für ein Erstellen von Abbildern mit geringen Anforderungen eine Systemklasse mit entsprechend geringen Anforderungen gewählt werden kann, kann für höhere Anforderungen beim Erstellen des Abbildes eine entsprechende Systemklasse und ein Kompatibilitätsmodus für diese gewählt werden, welche Werte der Komponenteneigenschaft der Systemkomponente zulässt, die außerhalb des Bereichs für die Systemklasse mit geringeren Anforderungen liegt.

Gemäß einigen Ausführungsformen ist das Bildgebungssystem als CT-Anlage (also insbesondere als Computertomografie-Anlage) ausgebildet.

Gemäß einigen Ausführungsformen ist das Bildgebungssystem als MR-Anlage (also insbesondere als Magnetresonanztomografie-Anlage) ausgebildet.

Bei einigen Ausführungsformen, bei welchen das Bildgebungssystem als MR-Anlage ausgebildet ist, ist die Systemkomponente eine Magnetfeldgradientenvorrichtung und die Komponenteneigenschaft eine Gradientenfeldstärke oder eine Anstiegsrate, die durch die Magnetfeldgradientenvorrichtung bei der Bestimmung des Abbildes erzeugt wird. Hierdurch lässt sich für einen trapezförmigen Schichtanregungspuls und eine etwaige Rephasierung bei unterschiedlichen MR-Anlagen, die jeweils im Kompatibilitätsmodus betrieben werden, ein vorgegebenes Gradientenmoment mit einem gleichen zeitlichen Gradientenfeldverlauf erzielen und somit die Konsistenz beim Bildeindruck steigern.

In einigen Varianten können die Komponenteneigenschaft oder weitere Komponenteneigenschaften sein:
- eine maximalen Gradientenamplitude;
- eine nominalen Gradientenamplitude;
- eine Gradientenanstiegsrate;
- eine Gradienten-Modellierung;
- eine maximale HF-Sendefeldstärke;
- eine mittlere HF-Sendefeldstärke;
- eine HF-Sende-Modellierung;
- eine Anzahl von HF-Sendekanälen;
- eine Anzahl von HF-Empfangskanälen
- eine (vorhandene/ausführbare) Softwarefunktion;
- eine (vorhandene/bereitgestellte/verwendbare) Hardware-Funktionalität wie etwa eine 3-D-Kamera zur Bewegungserfassung und/oder zur Korrektur von Bewegungen, ein (Nicht-) Vorhandensein von bestimmten HF-Empfangsspulen oder ein (Nicht-) Vorhandensein von bestimmten Sendespulen;
- ein Patientenmodell für SAR-Limitierung;
- ein Patientenmodell für Nervenstimulation; oder
- eine Kombination daraus.

Bei einigen Varianten kann mittels der maximalen Gradientenamplitude vorteilhaft für Diffusionsgradientenpulse - die oft in "physikalischen" Koordinaten und nicht in "logischen" Koordinaten, die an die Bildorientierung angelehnt sind - eine Gradientenfeldstärke in einem Messprotokoll festgelegt werden.

Bei einigen Varianten kann mittels der nominalen Gradientenamplitude vorteilhaft eine Gradientenfeldstärke in einem Messprotokoll in "logischen" Koordinaten festgelegt werden.

Bei einigen Varianten kann eine Beschreibung von Gradientenfeldstärken mittels der Gradienten-Modellierung vorteilhaft die Leistungsfähigkeit eines solchen Bildgebungssystems komplex beschreiben, etwa wie groß die Amplitude von Gradientenpulsen bei einer bestimmten zeitlichen Abfolge sein darf, damit sie von den Verstärkern generiert werden können. Dies wird etwa bei Messsequenzen, die im Grenzbereich der Hardware operieren (z.B. Diffusionsmessungen) verwendet.

Bei einigen Varianten ermöglicht die HF-Sende-Modellierung eine komplexere Beschreibung der Leistungsfähigkeit (die Betrachtung der mittleren HF-Sendefeldstärke kann eine vereinfachte Modellierung ermöglichen).

Bei einigen Varianten ermöglicht eine größere Anzahl an Sendekanälen etwa höhere Beschleunigungsfaktoren bei Anwendungen mit "paralleler Transmission" (pTx) (dies betrifft betriff beispielsweise zwei- oder dreidimensional räumlich-selektive Anregungspulse).

Bei einigen Varianten ermöglicht eine größere Anzahl an Empfangskanälen höhere Beschleunigungsfaktoren bei der parallelen Bildgebung (z.B. GRAPPA, SENSE, CAIPIRINHA).

So kann etwa bei einigen Varianten eine Software-Version zusätzliche Funktionen aufweisen z.B. kann eine erste Version keine parallele Bildgebung für die 3D-Bildgebung unterstützen, eine Folgeversion GRAPPA-Beschleunigung in einer Dimension und eine weitere Folgeversion GRAPPA- (oder CAIPIRINHA-) Beschleunigung in zwei Dimensionen, wobei im Kompatibilitätsmodus solche Funktionen zugelassen werden, welche auf allen Bildgebungssystemen (dieser Systemklasse) verfügbar/möglich sind.

Bei einigen Varianten können mittels eines Patientenmodells für Nervenstimulation vorteilhaft die Änderungsraten der Gradientenfelder (etwa aufgrund von regulatorischer Beschränkung zur Vermeidung ausgeprägter Stimulationen der peripheren Nerven), wobei beispielsweise das Design der Gradientenspule eine Rolle spielt, oder können vorteilhaft die Anstiegsraten bestimmter Gradientenpulse limitiert werden.

Gemäß einigen Ausführungsformen ist die Steuerungskomponente eingerichtet, um ein Messprotokoll für einen Kompatibilitätsmodus - etwa einen Kompatibilitätsmodus für eine erste Systemklasse - in ein Messprotokoll für einen anderen Kompatibilitätsmodus - etwa einen Kompatibilitätsmodus für eine zweite Systemklasse - zu konvertieren oder umgekehrt. Hierdurch lassen sich Messprotokolle automatisch mit verschiedenen Kompatibilitätsmodi verwenden, wodurch sich die Effizienz steigern lässt. Bei einigen Varianten, bei welchen der Überlappungsbereich und der zweite Wertebereich disjunkt sind, kann das automatische Konvertieren von Messprotokollen oder - alternativ oder zusätzlich - ein Bereitstellen von entsprechenden Messprotokollen für den jeweils anderen Kompatibilitätsmodus besonders vorteilhaft sein, da die Bildgebungssysteme der ersten Systemklasse und die Bildgebungssysteme der zweite Systemklasse bezüglich der Werte ihrer jeweiligen Komponenteneigenschaft nicht in einem überlappenden Bereich, also mit gleichen Werten, betrieben werden können. Durch die (automatische) Konvertierung oder etwaig manuell angepasste Messprotokolle können diese Messprotokolle jedoch insoweit optimiert werden, um einen zumindest weitgehend gleichen Bildeindruck bei einem über das jeweilige Messprotokoll erstellten Abbild zu erzielen.

Gemäß einigen Ausführungsformen hat die Systemkomponente eine weitere Komponenteneigenschaft deren Werte sich nicht mit Werten einer entsprechenden weiteren Komponenteneigenschaft der Systemkomponente des anderen Bildgebungssystems harmonisieren lassen, d.h. insbesondere keinen gemeinsamen Überlappungsbereich aufweisen. Dabei ist die Steuerungskomponente ein einem hybriden Kompatibilitätsmodus als der Kompatibilitätsmodus oder als ein weiterer Kompatibilitätsmodus betreibbar. Die Steuerungskomponente ist eingerichtet, bei der Erstellung des Abbildes ein hybrides Messprotokoll auszuführen, welches wenigstens je ein (Unter-) Messprotokoll für die Werte der weiteren Systemkomponente des Bildgebungssystems und ein (Unter-) Messprotokoll für die Werte der weiteren Systemkomponente des anderen Bildgebungssystems aufweist, und dazu das jeweils geeignete Messprotokoll (also das (Unter-) Messprotokoll für die Werte der weiteren Systemkomponente des Bildgebungssystems) auszuwählen und auszuführen. Dabei sind die beiden (Unter-) Messprotokolle so aufeinander abgestimmt, dass diese bei ihrer Ausführung einen weitgehend gleichen Bildeindruck erzielen, obwohl die einstellbaren Werte für die jeweilige weitere Systemkomponente verschieden sind.

Ein zweiter Aspekt der Erfindung betrifft ein medizinisches Bildgebungssystem mit einem ersten Bildgebungssystem gemäß dem ersten Aspekt der Erfindung und einem zweiten Bildgebungssystem gemäß dem ersten Aspekt der Erfindung. Dabei sind das erste und das zweite Bildgebungssystem jeweils zur Bestimmung eines Abbildes eines Patienten als Untersuchungsobjekt ausgebildet. Zudem ist der durch das erste Bildgebungssystem bedingte erste Wertebereich verschieden von dem anderen durch das zweite Bildgebungssystem bedingten Wertebereich, wobei der erste Wertebereich und der andere Wertebereich einen Überlappungsbereich aufweisen. Dabei kann die Steuerungskomponente des ersten und des zweiten Bildgebungssystems jeweils in einem Kompatibilitätsmodus betreibbar sein und in diesem eingerichtet sein, nur Werte in diesem Überlappungsbereich zuzulassen.

Die bereits vorausgehend genannten möglichen Vorteile, Ausführungsformen oder Varianten des ersten Aspekts der Erfindung gelten entsprechend auch für das medizinische Bildgebungssystem.

Ein dritter Aspekt Erfindung betrifft ein Verfahren zur Erstellung eines Abbildes von einem Untersuchungsobjekt mittels eines Bildgebungssystems, insbesondere aus einem Verband/einer Flotte von mehreren unterschiedlichen Bildgebungssystemen, insbesondere einem medizinischen Bildgebungssystem. Das Bildgebungssystem weist eine Systemkomponente auf, die eine Komponenteneigenschaft hat, welche bei Erstellung des Abbildes einen Wert in einem durch das Bildgebungssystem bedingten ersten Wertebereich annehmen kann. Zudem weist eine entsprechende Systemkomponente eines anderen Bildgebungssystems, insbesondere aus dem Verband/der Flotte, eine entsprechende Komponenteneigenschaft auf, deren Werte einen anderen Wertebereich einnehmen können, wobei der andere Wertebereich mit dem ersten Wertebereich einen Überlappungsbereich aufweist. Das Verfahren weist auf: Aktivieren eines Kompatibilitätsmodus; und Erstellen des Abbildes, wobei für die Komponenteneigenschaft der Systemkomponente im Kompatibilitätsmodus nur Werte zugelassen werden, die in dem Überlappungsbereich liegen.

In einigen Varianten kann das Bildgebungssystem ein Bildgebungssystem gemäß dem ersten Aspekt der Erfindung sein. Auch kann in einigen Varianten der Verband mehrere Bildgebungssysteme gemäß dem ersten Aspekt der Erfindung aufweisen. Zusätzlich oder alternativ kann in einigen Varianten der Verband oder das medizinische Bildgebungssystem ein medizinisches Bildgebungssystem gemäß dem zweiten Aspekt der Erfindung sein.

Auch wird das Verfahren in einigen Varianten auf dem Bildgebungssystem ausgeführt und eine Steuerungskomponente durch Aktivieren des Kompatibilitätsmodus in diesem betrieben.

Die bereits voraus genannten möglichen Vorteile, Ausführungsformen oder Varianten der vorhergehenden Aspekte der Erfindung gelten entsprechend auch für das erfindungsgemäße Verfahren.

Ein vierter Aspekt der Erfindung betrifft eine Verwendung eines Bildgebungssystems gemäß dem ersten Aspekt der Erfindung, eines medizinischen Bildgebungssystems gemäß dem zweiten Aspekt der Erfindung oder eines Verfahrens gemäß dem dritten Aspekt der Erfindung zum Erstellen von mehreren Abbildern von einem oder von mehreren Untersuchungsobjekten, wobei die Abbilder mittels einer automatischen Bildauswertung verarbeitet und/oder als Trainingsdaten für maschinelles Lernen verwendet werden. Ein Vorteil der Verwendung der vorhergehenden Aspekte der Erfindung mit einer automatischen Bildauswertung oder als Trainingsdaten für maschinelles Lernen kann insbesondere darin liegen, dass durch den Kompatibilitätsmodus ein gleichbleibender Bildeindruck gewährleistet wird, wodurch sich bei der automatischen Bildauswertung eine höhere Genauigkeit erzielen lässt bzw. beim Training im maschinellen Lernen eine schnellere Konvergenz und/oder eine robustere Modellierung ermöglicht wird.

Die bereits vorausgehend genannten möglichen Vorteile, Ausführungsformen oder Varianten der vorhergehenden Aspekte der Erfindung gelten entsprechend auch für die erfindungsgemäße Verwendung.

Bei einigen Ausführungsformen werden im Verfahren zum maschinellen Lernen, insbesondere mittels künstlicher neuronaler Netze, als Trainingsdaten mehrere Abbilder mittels wenigstens eines Bildgebungssystem im Kompatibilitätsmodus erstellt und basierend darauf ein maschinelles Lernen durchgeführt. Durch den konsistenten Bildeindruck lassen sich eines oder mehrere Charakteristika, welche durch das maschinelle Lernen erkannt werden sollen, einfacher trainieren und/oder höhere Erkennungsgenauigkeiten erzielen. Auch wird es ermöglicht, derartige Trainingsdaten anhand einer größeren Anzahl an entsprechenden Bildgebungssystemen, welche zwar einen gemeinsamen Kompatibilitätsmodus haben - aber sich etwaig ansonsten voneinander unterscheiden, zu gewinnen. Hierdurch lassen sich die Bildgebungssysteme besser auslasten und/oder Trainingsdaten in einem größeren Umfang oder schneller gewinnen, als es mit nur einem Typ an Bildgebungssystem - welcher also dadurch, dass alle Bildgebungssysteme dieses Typs gleich sind, konsistente Bildwirkungen liefern würde - möglich wäre.

Verfahren, Vorrichtungen, Systeme und Verwendungen nach Ausführungsformen sowie nachfolgenden Ausführungsbeispielen ermöglichen insbesondere das Erstellen von Abbildern von einem oder von mehreren Untersuchungsobjekten und können dabei insbesondere einen konsistenten, also insbesondere gleichen, Bildeindruck ermöglichen. Dabei können insbesondere einige Ausführungsformen und/oder einige Ausführungsbeispiele des Verfahrens zumindest teilweise automatisiert ausgeführt werden. Dazu kann insbesondere in einigen Ausführungsformen und/oder Ausführungsbeispielen eine Vorrichtung oder ein System oder ein Teil davon eingerichtet sein, um Verfahrensteile automatisch auszuführen, bzw. können Verfahrensteile mittels dieser Vorrichtungen, Systeme oder Teilen davon automatisch ausgeführt werden.

Im Sinne der Erfindung ist unter "automatisch" zumindest zu verstehen, dass ein Teil eines Verfahrens, insbesondere ein Verfahrensschritts oder ein Vorgang im Verfahren, und/oder eine Funktionalität einer Vorrichtung/eines Systems ohne menschlichen Eingriff ausgeführt werden kann.

Weitere Vorteile, Merkmale und Anwendungsmöglichkeiten ergeben sich aus der nachfolgenden detaillierten Beschreibung von Ausführungsbeispielen und/oder aus den Figuren.

Die Erfindung wird nachfolgend unter Bezugnahme auf die Figuren anhand vorteilhafter Ausführungsbeispiele näher erläutert. Gleiche Elemente oder Bauteile der Ausführungsbeispiele werden im Wesentlichen durch gleiche Bezugszeichen gekennzeichnet, falls dies nicht anders beschrieben wird oder sich nicht anders aus dem Kontext ergibt.

Hierzu zeigen, teilweise schematisiert:
Fig. 1: eine MR-Anlage nach einer Ausführungsform;
Fig. 2: ein Flussdiagram eines Verfahrens zur Erstellung eines Abbildes von einem Untersuchungsobjekt in einem Leistungsmodus nach einer Ausführungsform; und
Fig. 3: ein Flussdiagram eines Verfahrens zur Erstellung eines Abbildes von einem Untersuchungsobjekt in einem Kompatibilitätsmodus nach einer Ausführungsform.

Die Figuren sind schematische Darstellungen verschiedener Ausführungsformen und/oder Ausführungsbeispiele der vorliegenden Erfindung. In den Figuren dargestellte Elemente und/oder Bauteile sind nicht notwendigerweise maßstabsgetreu dargestellt. Vielmehr sind die verschiedenen in den Figuren dargestellten Elemente und/oder Bauteile derart wiedergegeben, dass ihre Funktion und/oder ihr Zweck dem Fachmann verständlich werden.

In den Figuren dargestellte Verbindungen und Kopplungen zwischen funktionellen Einheiten und Elementen können auch als indirekte Verbindungen oder Kopplungen implementiert werden. Insbesondere können Datenverbindungen drahtgebunden oder drahtlos, also insbesondere als Funkverbindungen ausgebildet sein. Auch können bestimmte Verbindungen, etwa elektrische Verbindungen, etwa zur Energieversorgung, der Übersichtlichkeit halber nicht dargestellt sein.

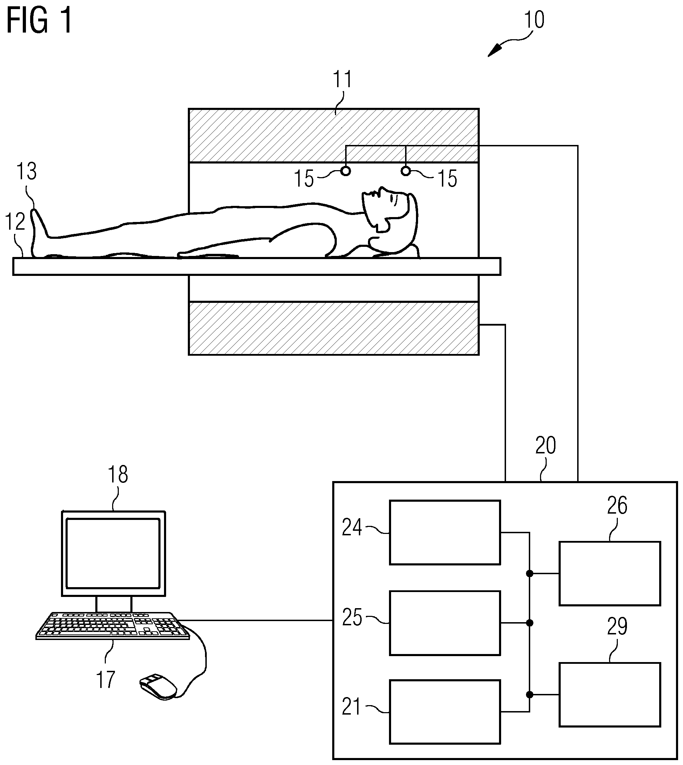

In Fig. 1 ist ein Bildgebungssystem 10 nach einer Ausführungsform der vorliegenden Erfindung schematisch dargestellt. Hierbei wird die Erfindung im Folgenden insbesondere anhand eines als MR-Anlage ausgebildeten Bildgebungssystems erläutert. Neben den Ausführungsbeispielen anhand der MR-Anlage offenbart die vorliegende Beschreibung dem Fachmann auch, wie er die Erfindung bei anderen Bildgebungssystemen wie etwa einer CT-Anlage oder einem Ultraschallgerät ausführen kann, wobei er spezifische Details der MR-Anlage durch entsprechende Details, also insbesondere Komponenten und/oder Funktionen oder Vorgänge, bzgl. der anderen Bildgebungssysteme ersetzt.

In einem Ausführungsbeispiel weist die MR-Anlage 10 einen Magneten 11 zur Erzeugung eines Polarisationsfeldes B0 auf, wobei eine auf einer Liege 12 angeordnete Untersuchungsperson 13 - als Untersuchungsobjekt - in den Magneten 11 gefahren wird, um dort ortskodierte Magnetresonanzsignale aus der Untersuchungsperson 13 aufzunehmen. Die zur Signalaufnahme verwendeten Spulen wie eine Ganzkörperspule oder Lokalspulen sind aus Übersichtlichkeitsgründen nicht dargestellt. Die Erfindung kann bei der sogenannten parallelen Bildgebung Anwendung finden, bei der die MR-Signale gleichzeitig mit mehreren Lokalspulen, einem Spulenarray von Lokalspulen, aufgenommen werden. Durch Einstrahlen von Hochfrequenzpulsen und Schalten von Magnetfeldgradienten mittels Gradientenspulen 15, insbesondere HF-Spulen als Sendespulen und/oder als Empfangsspulen, kann die durch das Polarisationsfeld B0 erzeugte Magnetisierung aus der Gleichgewichtslage ausgelenkt und ortskodiert werden, und die sich ergebende Magnetisierung wird von den Empfangsspulen detektiert. Wie durch Einstrahlen der HF-Pulse und durch Schalten von Magnetfeldgradienten in verschiedenen Kombinationen und Reihenfolgen MR-Bilder erzeugt werden können, ist dem Fachmann grundsätzlich bekannt und wird hier nicht näher erläutert.

Die MR-Anlage 10 weist weiterhin eine Steuerungskomponente 20 auf, die zur Steuerung der MR-Anlage verwendet werden kann. Die Steuerungskomponente 20 weist eine Gradientensteuereinheit 25 zur Steuerung und Schaltung der notwendigen Magnetfeldgradienten mittels der Gradientenspulen 15 auf. Eine HF-Steuereinheit 24 ist für die Steuerung und Generierung der HF-Pulse zur Auslenkung der Magnetisierung vorgesehen. Eine Bildsequenzsteuerung 26 steuert die Abfolge der Magnetfeldgradienten, der Signaldetektion und HF-Pulse und damit indirekt die Gradientensteuereinheit 25, die Empfangsspulen und die HF-Steuereinheit 24. Über eine Eingabeeinheit 17 kann eine Bedienperson die MR-Anlage steuern und auf einer Anzeigeeinheit 18 können MR-Bilder und sonstige zur Steuerung notwendige Informationen angezeigt werden. Eine Recheneinheit 29 mit mindestens einer Prozessoreinheit (nicht gezeigt) ist vorgesehen zur Steuerung der verschiedenen Einheiten in der Steuerungskomponente 20. Weiterhin ist eine Speichereinheit 21 vorgesehen, in der beispielsweise Programmmodule bzw. Programme abgespeichert werden können, die, wenn sie von der Recheneinheit 29 bzw. ihrer Prozessoreinheit ausgeführt werden, den Ablauf der MR-Anlage steuern können. Dabei sind in einigen Varianten auch Messprotokolle in der Speichereinheit 21 gespeichert, welche wiederum den Ablauf der MR-Anlage steuern können, wobei diese etwaige Programmmodule oder Programme nutzen können. Die Steuerungskomponente 20, insbesondere die Recheneinheit 29, ist, wie nachfolgend erläutert, ausgebildet, um in einem Leistungsmodus und in einem Kompatibilitätsmodus betrieben zu werden, wobei die Steuerung von dem jeweiligen Betriebsmodus abhängig ist.

Im Folgenden werden der Kompatibilitätsmodus und der Leistungsmodus anhand dreier MR-Anlagen erläutert. Dabei kann eine der MR-Anlagen, etwa die erste MR-Anlage, als Ausführungsbeispiel des erfindungsgemäßen Bildgebungssystems ausgebildet sein, während die übrigen MR-Anlagen, etwa die zweite und die dritte MR-Anlage, jeweils als ein anderes Bildgebungssystem ausgebildet sein können. In einigen Varianten können die drei MR-Anlagen auch als ein Ausführungsbeispiel des medizinischen Bildgebungssystems ausgebildet sein, wobei dieses zusätzlich zum ersten und zweiten Bildgebungssystem ein drittes Bildgebungssystem, nämlich insbesondere die dritte MR-Anlage aufweist.

Die erste MR-Anlage weist als Systemkomponente eine Magnetfeldgradientenvorrichtung mit den Gradientenspulen 15 und der Gradientensteuereinheit 25 auf. Die mit dieser Magnetfeldgradientenvorrichtung (maximal) erreichbare Magnetfeldstärke beträgt 60 mT/m und die (maximal) erreichbare Anstiegsrate beträgt 200 mT/m/ms. Entsprechend sind die Magnetfeldstärke und die Anstiegsrate jeweils eine Komponenteneigenschaft der Systemkomponente, also hier der Magnetfeldgradientenvorrichtung, die jeweils Werte in einem ersten Wertbereich annehmen können, welcher durch die MR-Anlage und insbesondere durch die Magnetfeldgradientenvorrichtung selbst bedingt ist.

Auch die zweite MR-Anlage weist eine entsprechende Magnetfeldgradientenvorrichtung auf, wobei die erreichbare Magnetfeldstärke 45 mT/m und die erreichbare Anstiegsrate 100 mT/m/ms beträgt.

Schließlich weist auch die dritte MR-Anlage eine entsprechende Magnetfeldgradientenvorrichtung auf, wobei die erreichbare Magnetfeldstärke 30 mT/m und die erreichbare Anstiegsrate 100 mT/m/ms beträgt.

In einem Messprotokoll zur Erstellung eines Abbildes von der Untersuchungsperson 13 ist ein trapezförmiger Schichtanregungspuls festgelegt, wobei für diesen die Gradientenamplitude 20 mT/m und die Dachdauer 3 ms beträgt. Für einen folgenden trapezförmigen Rephasierungpuls im Messprotokoll muss das ab der Mitte des Schichtanregungspulses aufgebaute nullte Gradientenmoment vollständig rephasiert werden.

Zunächst wird der Ablauf bei den MR-Anlagen beschrieben, wenn diese im jeweiligen Leistungsmodus betrieben werden, so dass anlagenspezifische Optimierungen im Messprotokoll bzw. beim Ausführen des Messprotokolls verwendet werden.

Bei der ersten MR-Anlage läuft in ihrem Leistungsmodus Folgendes ab:
- Anstieg auf 20mT/m mit 200mT/m/ms in 0.1ms, 3ms Dachdauer, 0.1ms Abfallzeit; und
- Rephasierung des Moments von (20mT/m * 3ms) / 2 + 10mT/m * 0.1ms = 31mT/m *ms mit einem Gradientenpuls der Amplitude 60mT/m, Rampenzeit 0.3ms und Dachdauer 0.22ms;
woraus sich eine Gesamtdauer von 0.1ms + 3.0ms + 0.1ms + 0.3ms + 0.22ms + 0.3ms = 4.02ms ergibt.

Bei der zweiten MR-Anlage läuft in ihrem Leistungsmodus Folgendes ab:
- Anstieg auf 20mT/m mit 100mT/m/ms in 0.2ms, 3ms Dachdauer, 0.2ms Abfallzeit; und
- Rephasierung des Moments von (20mT/m * 3ms) / 2 + 10mT/m * 0.2ms = 32mT/m *ms mit einem Gradientenpuls der Amplitude 45mT/m, Rampenzeit 0.45ms und Dachdauer 0.26ms;
woraus sich eine Gesamtdauer von 0.2ms + 3.0ms + 0.2ms + 0.45ms + 0.26ms + 0.45ms = 4.56ms ergibt.

Bei der dritten MR-Anlage läuft in ihrem Leistungsmodus Folgendes ab:
- Anstieg auf 20mT/m mit 100mT/m/ms in 0.2ms, 3ms Dachdauer, 0.2ms Abfallzeit; und
- Rephasierung des Moments von (20mT/m * 3ms) / 2 + 10mT/m * 0.2ms = 32mT/m * ms mit einem Gradientenpuls der Amplitude 30mT/m, Rampenzeit 0.3ms und Dachdauer 0.77ms;
woraus sich eine Gesamtdauer von 0.2ms + 3.0ms + 0.2ms + 0.3ms + 0.77ms + 0.3ms = 4.77ms ergibt.

Nun wird der Ablauf im Kompatibilitätsmodus beschrieben. Dabei weisen die erreichbaren Magnetfeldstärken der Systemkomponenten (also der Magnetfeldgradientenvorrichtung der drei MR-Anlagen) als Überlappungsbereich eine (maximal) erreichbare Magnetfeldstärke von 30 mT/m auf. Weiterhin weisen die erreichbaren Anstiegsraten der Systemkomponenten (also der Magnetfeldgradientenvorrichtung der drei MR-Anlagen) als Überlappungsbereich eine (maximal) erreichbare Anstiegsrate von 100 mT/m/ms auf.

Somit ergibt sich im Kompatibilitätsmodus für alle drei MR-Anlagen der gleiche Ablauf, nämlich:
- Anstieg auf 20mT/m mit 100mT/m/ms in 0.2ms, 3ms Dachdauer, 0.2ms Abfallzeit; und
- Rephasierung des Moments von (20mT/m * 3ms) / 2 + 10mT/m * 0.2ms = 32mT/m *ms mit einem Gradientenpuls der Amplitude 30mT/m, Rampenzeit 0.3ms und Dachdauer 0.77ms;
woraus sich eine Gesamtdauer von 0.2ms + 3.0ms + 0.2ms + 0.3ms + 0.77ms + 0.3ms = 4.77ms ergibt.

Zwar verlängert sich gegenüber dem Leistungsmodus die Gesamtdauer der Anregung bei der ersten MR-Anlage um 19 % und bei der zweiten MR-Anlage um 5 %, jedoch lässt sich durch den gleichen Ablauf ein gleicher Bildeindruck erzielen. Der Vorteil des gleichen Bildeindruck kann etwa für eine Diagnose der Untersuchungsperson 13, eine geringfügig verlängerte Aufnahmedauer, welche aus der verlängerten Gesamtdauer der Anregung resultieren könnte, überwiegen. Insbesondere ist im medizinischen Alltag neben der Aufnahmedauer auch weitere Zeit für eine Diagnose der Untersuchungsperson nötig, etwa zur Lagerung der Untersuchungsperson, zur Aufklärung, ggf. zur Kontrastmittelinjektion, zur Messplanung und/oder für Justagen. Damit kann in einigen Fällen die geringfügig verlängerte Aufnahmedauer nicht weiter ins Gewicht fallen. Dies kann insbesondere bei folgenden Anwendungen der Fall sein: Erstellung von Abbildern der Wirbelsäule, anatomischen Aufnahmen, onkologischen Aufnahmen und/oder kontrastmittelgestützten MRT-Aufnahmen, sofern diese nicht eine Dynamik des Kontrastmittels erfassen sollen/müssen.

Hingegen kann das Betreiben der MR-Anlage im Leistungsmodus besonders für spezielle Anwendungen mit hohen Hardware-Anforderungen vorteilhaft sein, so dass die volle Leistungsfähigkeit der jeweiligen MR-Anlage ausgeschöpft werden kann. Derartige Anwendungen können etwa eine Diffusions-Tensor-Bildgebung, eine Ganzkörper-Diffusionsbildgebung oder Messungen mit hoher zeitlicher Auflösung am Herzen oder nach Verabreichung von Kontrastmitteln (insbesondere zur Erfassung einer Dynamik bei der Kontrastmittelverabreichung) sein. Hierbei lässt sich durch Ausschöpfen der Leistungsfähigkeit der MR-Anlage die diagnostische Qualität steigern.

Auch können im medizinischen Alltag die unterschiedlichen MR-Anlagen in unterschiedliche Systemklassen unterteilt werden mit jeweils unterschiedlicher Leistungsfähigkeit. So können etwa (fast) alle MR-Anlagen im Kompatibilitätsmodus für eine zweite Systemklasse mit den geringsten Anforderungen an die Leistungsfähigkeit betreibbar sein, wobei bereits in diesem Kompatibilitätsmodus viele grundlegende diagnostische Anwendungen, also insbesondere Routine-Anwendungen wie etwa anatomische Bildgebung mit T1-, T2- oder PD-Kontrast am Gehirn, an der Wirbelsäule oder an großen Gelenken - etwa mittels TSE-Techniken - oder auch Aufnahmen in der Onkologie durchgeführt werden können. Hingegen kann ein Teil der MR-Anlagen mit höherer Leistungsfähigkeit einer ersten Systemklasse (zusätzlich) zugeordnet sein und es können diese MR-Anlagen in einem Kompatibilitätsmodus für die erste Systemklasse betreibbar sein, sodass diese in dem Kompatibilitätsmodus für die erste Systemklasse spezielle Anwendungen mit hohen Hardware-Anforderungen ermöglichen.

Auch kann die MR-Anlage eingerichtet sein, um bei Aktivierung des Kompatibilitätsmodus Abhängigkeiten und Einschränkungen von Messparametern automatisch zu berücksichtigen. So können etwa Optionen, die nur mit bestimmten Lizenzen oder nur mit bestimmten Softwareversionen - die bestimmte in Software implementierte Funktionen bereitstellen - und damit nicht auf jeder MR-Anlage (auch etwa innerhalb einer Systemklasse) verfügbar sind, automatisch abgeschaltet und vorzugsweise durch auf der jeweiligen MR-Anlage verfügbare Alternativen ersetzt werden. So könnte etwa eine zweidimensionale CAIPIRINHA-Unterabtastung (Controlled Aliasing in Parallel Imaging Results in Higher Acceleration) durch eine eindimensionale GRAPPA-Unterabtastung (Generalized Autocalibrating Partially Parallel Acquisition) ersetzt werden. Hierbei könnten Timing-Konflikte durch Verlängerung von Zeitparametern wie etwa TE oder TR etc. automatisch aufgelöst werden. Auch könnten Optionen, die für das Erreichen eines konsistenten Bildeindrucks wichtig sind, automatisch angepasst werden wie etwa eine Bildnormalisierung oder eine Bildfilterung.

Allerdings lassen sich manche Komponenteneigenschaften von Systemkomponenten von Bildgebungssystemen, insbesondere MR-Anlagen nicht durch einen (einfachen) Kompatibilitätsmodus harmonisieren. Bei MR-Anlagen betrifft dies etwa die Grundfeldstärke, wobei etwa MR-Anlagen mit einer Grundfeldstärke von 1,5 T gegenüber MR-Anlagen mit einer Grundfeldstärke von 3 T auch bei identischen Messprotokollen und entsprechenden Messabläufen/Messprotokollen Abbilder erzeugen, welche unterschiedliche Kontrasteigenschaften aufweisen und damit einen unterschiedlichen Bildeindruck bedingen. Für derartige Fälle kann man zwei angepasste Kompatibilitätsmodi oder einen (hybrid) Kompatibilitätsmodus mit zwei korrespondierenden Messprotokollen vorsehen, welche für die nichtharmonisierbare Komponenteneigenschaft derart optimiert sind, dass beim Ausführen dieser beiden optimierten Messprotokolle zum Erstellen jeweils eines Abbildes von der Untersuchungsperson 13 zumindest weitgehend ein gleicher Bildeindruck erzielt wird. Hierzu können verschiedene Parameter, welche etwa von der Grundfeldstärke abhängen, wie etwa Echozeiten TE, Repetitionszeiten TR, Inversionszeiten TI, aber auch die Anzahl notwendiger Mittelungen oder ggf. sogar vollkommen unterschiedliche Messtechniken bei den beiden Messprotokollen angepasst werden. Derartige Anpassungen können mittels automatischer Konvertierung, simulationsbasierten Optimierungen und/oder manuell durchgeführt werden. Dabei werden die beiden Messprotokolle für die beiden Kompatibilitätsmodi oder für den einen (hybriden) Kompatibilitätsmodus, welcher beide Messprotokolle ermöglicht, abgespeichert, so dass zumindest im jeweiligen Kompatibilitätsmodus oder mit dem jeweiligen Messprotokoll auf verschiedenen MR-Anlagen ein konsistenter Bildeindruck und bei dem jeweils anderen Messprotokoll zumindest ein weitgehend gleicher Bildeindruck erzielt wird und die Abweichungen der Bildeindrücke konsistent sind. Entsprechend können auch die beiden korrespondierenden Messprotokolle zu einem hybriden Messprotokoll zusammengefasst werden, aus welchen dann insbesondere das jeweils für den jeweiligen Kompatibilitätsmodus passende (Unter-) Messprotokoll ausgewählt wird. Speziell bei MR-Anlagen mit unterschiedlicher Grundfeldstärke kann etwa bei MR-Anlagen mit einer Grundfeldstärke von 1,5 T in dem einen Messprotokoll eine konventionelle Spin-Echo-Bildgebung durchgeführt werden, während bei MR-Anlagen mit einer Grundfeldstärke von 3 T korrespondierend dazu im korrespondierenden Messprotokoll eine Gradienten-Echo-Bildgebung mit Inversionspräparation durchgeführt wird. Ein Vorteil der beiden Kompatibilitätsmodi bzw. der korrespondierenden Messprotokolle kann insbesondere darin liegen, dass jeweils ein für die jeweilige MR-Anlage (etwa aus einer Flotte an MR-Anlagen in einem Klinikverbund) geeignetes Messprotokoll automatisch aus den gespeicherten (korrespondierenden) Messprotokollen ausgewählt und ausgeführt werden kann, was die Zuverlässigkeit bei der Erstellung eines Abbildes steigert und das Betreiben solcher Flotten oder Verbände an MR-Anlagen effizienter macht.

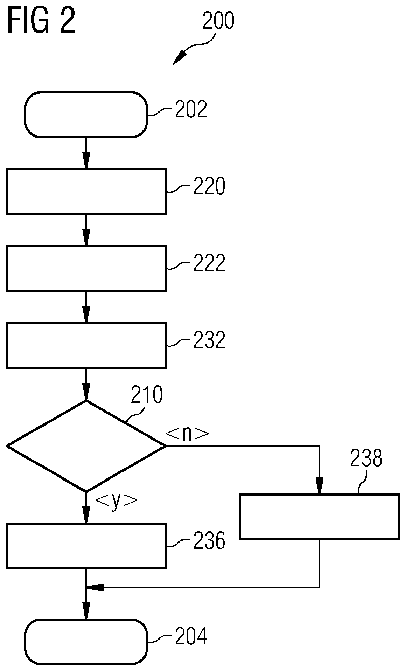

In Fig. 2 ist ein Flussdiagram eines Verfahrens 200 zur Erstellung eines Abbildes von einem Untersuchungsobjekt, etwa einer Untersuchungsperson, mittels eines Bildgebungssystems, welches in einem Leistungsmodus betrieben wird, nach einer Ausführungsform der vorliegenden Erfindung dargestellt. Das Bildgebungssystem weist eine Systemkomponente auf, die eine Komponenteneigenschaft hat, welche bei Erstellung des Abbildes einen Wert in einem durch das Bildgebungssystem bedingten ersten Wertebereich annehmen kann.

In einem Ausführungsbeispiel entspricht das Bildgebungssystem der bezüglich Fig. 1 beschriebenen MR-Anlage.

In einem Ausführungsbeispiel weist das Verfahren 200 die Verfahrensschritte 220, 222, 232, 236 und 238 sowie die Verfahrensbedingung 210 auf. Das Verfahren 200 beginnt bei dem Verfahrensstart 202 und endet bei dem Verfahrensende 204, wobei einer oder mehrere Verfahrensschritte, insbesondere eine Sequenz von Verfahrensschritten, und vorzugsweise das gesamte Verfahren wiederholt ausgeführt werden können.

Im Verfahrensschritt 220 wird der Leistungsmodus bei dem Bildgebungssystem, insbesondere der MR-Anlage, aktiviert, wobei in einigen Varianten dazu eine Steuerungskomponente des Bildgebungssystems in dem Leistungsmodus betrieben wird.

Im Verfahrensschritt 222 wird ein Messprotokoll für den Leistungsmodus geladen und dazu etwa aus einer Speichereinheit 21 ausgelesen.

Im Verfahrensschritt 232 wird überprüft, ob Werte für die Komponenteneigenschaft, innerhalb des ersten Wertebereichs liegen. So kann etwa bei der MR-Anlage überprüft werden, ob die einzustellenden Feldstärken oder Anstiegsraten die maximal erreichbare Feldstärke/Anstiegsrate nicht überschreiten.

Sofern die einzustellenden Werte im ersten Wertebereich liegen, wird gemäß der Verfahrensbedingung 210 - was dort durch <y> symbolisiert ist - der Verfahrensschritt 236 ausgeführt.

Im Verfahrensschritt 236 wird das Messprotokoll ausgeführt, werden die Werte der Komponenteneigenschaft entsprechend dem Messprotokoll eingestellt und so ein Abbild des Untersuchungsobjektes erstellt.

Andernfalls wird gemäß Verfahrensbedingung 210 - symbolisiert durch <n> - der Verfahrensschritt 238 ausgeführt.

Im Verfahrensschritt 238 wird ein Fehlersignal ausgegeben, welches angibt, dass einzustellende Werte außerhalb des (zulässigen) Wertebereichs liegen.

Durch das Verfahren, in welchem das Bildgebungssystem im Leistungsmodus betrieben wird, lässt sich vorteilhaft die Leistungsfähigkeit des Bildgebungssystems ausreizen, während sichergestellt wird, dass keine Werte außerhalb des ersten Wertebereichs und damit unzulässige Werte eingestellt werden.

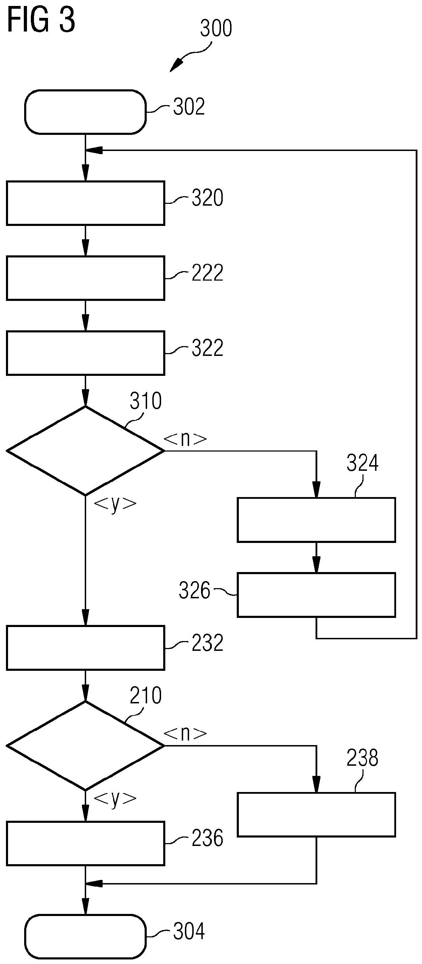

In Fig. 3 ist ein Flussdiagram eines Verfahrens 300 zur Erstellung eines Abbildes von einem Untersuchungsobjekt, etwa einer Untersuchungsperson 13, mittels eines Bildgebungssystems in einem Kompatibilitätsmodus nach einer Ausführungsform der vorliegenden Erfindung dargestellt. Das Bildgebungssystem weist eine Systemkomponente auf, die eine Komponenteneigenschaft hat, welche bei Erstellung des Abbildes einen Wert in einem durch das Bildgebungssystem bedingten ersten Wertebereich annehmen kann. Zudem weist eine entsprechende Systemkomponente eines anderen Bildgebungssystems eine entsprechende Komponenteneigenschaft auf, deren Werte einen anderen Wertebereich einnehmen können, wobei der andere Wertebereich mit dem ersten Wertebereich einen Überlappungsbereich aufweist.

In einem Ausführungsbeispiel entspricht das Bildgebungssystem der bezüglich Fig. 1 erläuterten MR-Anlage 10.

In einem Ausführungsbeispiel weist das Verfahren 300 die Verfahrensschritte 222, 232, 236, 238, 320, 322, 324 und 326 sowie die Verfahrensbedingungen 210 und 310 auf. Das Verfahren 300 beginnt bei dem Verfahrensstart 302 und endet bei dem Verfahrensende 304, wobei einer oder mehrere Verfahrensschritte, insbesondere eine Sequenz von Verfahrensschritten, und vorzugsweise das gesamte Verfahren wiederholt ausgeführt werden können.

Im Verfahrensschritt 320 wird der Kompatibilitätsmodus aktiviert. Dabei kann in einigen Varianten eine Steuerungskomponente des Bildgebungssystems, etwa die Steuerungskomponente 20 der MR-Anlage 10 gemäß Fig. 1, in dem Kompatibilitätsmodus betrieben werden.

Im Verfahrensschritt 222 wird ein Messprotokoll für den Kompatibilitätsmodus geladen.

Im Verfahrensschritt 322 wird überprüft, ob Werte für die Komponenteneigenschaft, welche beim Ausführen des Messprotokolls einzustellen sind, im Überlappungsbereich liegen.

Sofern dies nicht der Fall ist, wird gemäß Verfahrensbedingung 310 - was durch <n> symbolisiert ist - zunächst Verfahrensschritt 324 und dann Verfahrensschritt 326 ausgeführt und anschließend das Verfahren nochmals beginnend von Verfahrensschritt 320 ausgeführt. Dabei wird im Verfahrensschritt 324 das Messprotokoll so konvertiert, dass keine Werte außerhalb des Überlappungsbereichs mehr auftreten und im Verfahrensschritt 326 dieses Messprotokoll gespeichert. Daraufhin kann nach Aktivierung des Kompatibilitätsmodus im Verfahrensschritt 320 das so konvertierte und gespeicherte Messprotokoll im Verfahrensschritt 222 geladen werden. Alternativ könnte das Verfahren nach der Konvertierung im Verfahrensschritt 324 oder nach der Speicherung im Verfahrensschritt 326 bei Verfahrensschritt 232 für das konvertierte Messprotokoll fortgesetzt werden. Auch könnte ggf. alternativ ein Fehlersignal ausgegeben und beendet werden oder das Verfahren könnte wie oben beschrieben fortgesetzt werden.

Sofern die einzustellenden Werte im Überlappungsbereich liegen, wird gemäß Verfahrensbedingung 310 - was dort durch ein <y> symbolisiert ist - der Verfahrensschritt 232 ausgeführt.

Dabei entspricht der übrige Ablauf ab Verfahrensschritt 232 dem Ablauf im Verfahren 200 bezüglich Fig. 2.

Ein Vorteil des Verfahrens 300, bei welchem das Abbild im Kompatibilitätsmodus erstellt wird, kann insbesondere darin liegen, dass die Komponenteneigenschaft nur Werte einnimmt, die im Überlappungsbereich liegen, wodurch sich auf unterschiedlichen Bildgebungssystemen, welche aber zumindest Werte für die entsprechende Komponenteneigenschaft im Überlappungsbereich ermöglichen, ein konsistenter Bildeindruck erzielen lässt.

Während Ausführungsbeispiele insbesondere unter Bezugnahme auf die Figuren detailliert beschrieben wurden, sei darauf hingewiesen, dass eine Vielzahl von Abwandlungen möglich ist. Außerdem sei darauf hingewiesen, dass es sich bei den exemplarischen Ausführungen lediglich um Beispiele handelt, die den Schutzbereich, die Anwendung und den Aufbau in keiner Weise einschränken sollen. Vielmehr wird dem Fachmann durch die vorausgehende Beschreibung ein Leitfaden für die Umsetzung von mindestens einem Ausführungsbeispiel gegeben, wobei diverse Abwandlungen, insbesondere alternative oder zusätzliche Merkmale und/oder Abwandlungen der Funktion und/oder Anordnung der beschriebenen Bestandteile, nach Wunsch des Fachmanns vorgenommen werden können, ohne dass dabei von dem in den angehängten Ansprüchen jeweils festgelegten Gegenstands sowie seiner rechtlichen Äquivalente abgewichen und/oder deren Schutzbereich verlassen wird.

## Patentansprüche

1. Bildgebungssystem (10) zur Erstellung eines Abbildes von einem Untersuchungsobjekt (13), aufweisend:
eine Systemkomponente (15,25), die eine Komponenteneigenschaft hat, welche bei Erstellung des Abbildes einen Wert in einem durch das Bildgebungssystem (10) bedingten ersten Wertebereich einnehmen kann;
wobei eine entsprechende Systemkomponente eines anderen Bildgebungssystems eine entsprechende Komponenteneigenschaft mit einem anderen Wertebereich aufweist, der mit dem ersten Wertebereich einen Überlappungsbereich aufweist;
eine Steuerungskomponente (20), die ausgebildet ist, eine Verwendung der Systemkomponente (15, 25) bei Erstellung des Abbildes zu steuern, wobei die Steuerungskomponente (20) in einem Kompatibilitätsmodus betreibbar ist, in dem
die Steuerungskomponente (20) eingerichtet ist, bei Erstellung des Abbildes nur Werte für die Komponenteneigenschaft der Systemkomponente (15, 25) zuzulassen, die in dem Überlappungsbereich liegen.

2. Bildgebungssystem (10) gemäß Anspruch 1, wobei:
die Steuerungskomponente (20) in dem Kompatibilitätsmodus oder in einem Leistungsmodus betreibbar ist; und
im Leistungsmodus die Steuerungskomponente (20) eingerichtet ist, bei Erstellung des Abbildes im Leistungsmodus Werte der Komponenteneigenschaft der Systemkomponente (15, 25) zuzulassen, die im ersten Wertebereich liegen aber außerhalb des Überlappungsbereichs.

3. Bildgebungssystem (10) gemäß Anspruch 2, wobei die Steuerungskomponente (20) weiterhin eingerichtet ist, im Leistungsmodus:
- ein Messprotokoll für die Erstellung des Abbildes für den Kompatibilitätsmodus in ein Messprotokoll für den Leistungsmodus zu konvertieren; und
- das konvertierte Messprotokoll auszuführen.

4. Bildgebungssystem (10) gemäß einem der vorhergehenden Ansprüche, wobei die Steuerungskomponente (20) weiterhin eingerichtet ist, zu überprüfen, ob bei Erstellung des Abbildes die einzustellenden Werte für die Komponenteneigenschaft im ersten Wertebereich liegen oder im Überlappungsbereich liegen; wobei die Erstellung des Abbildes in Abhängigkeit von einem Ergebnis der Überprüfung durchgeführt wird oder nicht oder, sofern die Werte zwar im ersten Wertebereich aber außerhalb des Überlappungsbereichs liegen, das Abbild in einem Leistungsmodus erstellt wird und ein Fehlersignal ausgegeben wird.

5. Bildgebungssystem (10) gemäß einem der Ansprüche 1 bis 3, wobei:
der Kompatibilitätsmodus ein erstes systemunabhängiges Bildgebungssystem mit einer systemunabhängigen Systemkomponente und einer systemunabhängigen Steuerungskomponente festlegt;
die systemunabhängige Systemkomponente eine Komponenteneigenschaft aufweist, welche mit der Komponenteneigenschaft der Systemkomponente (15,25) des Bildgebungssystems (10) korrespondiert und nur einen oder mehrere Werte in einem systemunabhängigen Wertebereich, der vollständig im Überlappungsbereich liegt, einnehmen kann;
die systemunabhängige Steuerungskomponente eingerichtet ist, ein Messprotokoll für den Kompatibilitätsmodus auszuführen und dabei den Wert der Komponenteneigenschaft der systemunabhängigen Systemkomponente auf einen oder mehrere Werte im systemunabhängigen Wertebereich einzustellen; und
die Steuerungskomponente (20) des Bildgebungssystems (10) eingerichtet ist:
- ein Messprotokoll für die Erstellung des Abbildes für den Kompatibilitätsmodus der systemunabhängigen Steuerungskomponente zur Ausführung bereitzustellen; und
- den Wert der Komponenteneigenschaft der Systemkomponente (15,25) anhand des Werts der Komponenteneigenschaft der systemunabhängigen Systemkomponente einzustellen.

6. Bildgebungssystem (10) gemäß einem der vorhergehenden Ansprüche, wobei:
die Steuerungskomponente (20) sowohl im Kompatibilitätsmodus für eine erste Systemklasse, die das Bildgebungssystem (10) und das andere Bildgebungssystem umfasst, als auch in einem Kompatibilitätsmodus für eine zweite Systemklasse, die zumindest ein noch ein anderes Bildgebungssystem umfasst, betreibbar ist; und
eine entsprechende Systemkomponente des noch einen anderen Bildgebungssystems eine entsprechende Komponenteneigenschaft mit einem zweiten Wertebereich aufweist, welcher durch das noch eine andere Bildgebungssystem bedingt ist, wobei die Komponenteneigenschaft der entsprechende Systemkomponente des noch einen anderen Bildgebungssystems nur einen oder mehrere Werte im zweiten Wertebereich einnehmen kann.

7. Bildgebungssystem (10) gemäß Anspruch 6, wobei:
die zweite Systemklasse das Bildgebungssystem (10) umfasst;
der zweite Wertebereich zumindest teilweise im Überlappungsbereich liegt und so einen zweiten Überlappungsbereich festlegt; und
die Steuerungskomponente (20) im Kompatibilitätsmodus für die zweite Systemklasse eingerichtet ist, bei Erstellung des Abbildes nur Werte für die Komponenteneigenschaft der Systemkomponente (15, 25) zuzulassen, die in dem zweiten Überlappungsbereich liegen.

8. Bildgebungssystem (10) gemäß Anspruch 6, wobei:
die zweite Systemklasse das Bildgebungssystem nicht umfasst;
der Überlappungsbereich und der zweite Wertebereich disjunkt sind; und
die Steuerungskomponente (20) im Kompatibilitätsmodus für die zweite Systemklasse eingerichtet ist, bei Erstellung des Abbildes Werte für die Komponenteneigenschaft, welche im zweiten Wertebereich aber außerhalb des Überlappungsbereichs liegen, in solche Werte zu konvertieren, die im Überlappungsbereich liegen.

9. Bildgebungssystem (10) gemäß Anspruch 6, wobei:
die zweite Systemklasse das Bildgebungssystem (10) umfasst, aber nicht das andere Bildgebungssystem umfasst;
der Überlappungsbereich und der zweite Wertebereich disjunkt sind;
der erste Wertebereich und der zweite Wertebereich zumindest teilweise überlappen und so einen zweiten Überlappungsbereich festlegen; und
die Steuerungskomponente (20) im Kompatibilitätsmodus für die zweite Systemklasse eingerichtet ist, bei Erstellung des Abbildes nur Werte für die Komponenteneigenschaft der Systemkomponente (15, 25) zuzulassen, die in dem zweiten Überlappungsbereich liegen.

10. Bildgebungssystem (10) gemäß einem der vorhergehenden Ansprüche, welches als CT-Anlage ausgebildet ist.

11. Bildgebungssystem (10) gemäß einem der Ansprüche 1 bis 10, welches als MR-Anlage ausgebildet ist, wobei die Systemkomponente eine Magnetfeldgradientenvorrichtung ist und die Komponenteneigenschaft eine Gradientenfeldstärke oder eine Anstiegsrate, die durch die Magnetfeldgradientenvorrichtung bei Bestimmung des Abbildes erzeugt wird, ist.

12. Medizinisches Bildgebungssystem mit einem ersten Bildgebungssystem (10) gemäß einem der vorhergehenden Ansprüche und einem zweiten Bildgebungssystem gemäß einem der vorhergehenden Ansprüche, wobei:
der durch das erste Bildgebungssystem (10) bedingte erste Wertebereich verschieden von dem anderen durch das zweite Bildgebungssystem bedingten Wertebereich ist; und
der erste Wertebereich und der andere Wertebereich den Überlappungsbereich aufweisen.

13. Verfahren zur Erstellung (300) eines Abbildes von einem Untersuchungsobjekt mittels eines Bildgebungssystems, welches eine Systemkomponente aufweist, die eine Komponenteneigenschaft hat, welche bei Erstellung des Abbildes einen Wert in einem durch das Bildgebungssystem bedingten ersten Wertebereich annehmen kann, wobei eine entsprechende Systemkomponente eines anderen Bildgebungssystems eine entsprechende Komponenteneigenschaft aufweist, deren Werte einen anderen Wertebereich einnehmen können, und wobei der andere Wertebereich mit dem ersten Wertebereich einen Überlappungsbereich aufweist; wobei das Verfahren (300) aufweist:
- (320) Aktivieren eines Kompatibilitätsmodus; und
- (236) Erstellen des Abbildes, wobei für die Komponenteneigenschaft der Systemkomponente im Kompatibilitätsmodus nur Werte zugelassen werden (310, 322), die in dem Überlappungsbereich liegen.

14. Verwendung eines Bildgebungsgeräts (10) gemäß einem der Ansprüche 1 bis 11, eines medizinischen Bildgebungssystems gemäß Anspruch 12 oder eines Verfahrens (300) gemäß Anspruch 13 zum Erstellen von mehreren Abbildern von einem Untersuchungsobjekt, wobei die Abbilder mittels einer automatischen Bildauswertung verarbeitet oder als Trainingsdaten für maschinelles Lernen verwendet werden.
